# EUROPEAN PATENT SPECIFICATION

(11) **EP 2 464 655 B1**
(45) Date of publication and mention of the grant of the patent: **15.02.2017**
(21) Application number: 10808035.9
(22) Date of filing: 09.08.2010
(51) Int. Cl.: C07K 1/20, C07K 7/64, C07K 7/16, C07K 14/62, C07K 14/46, C07K 7/06

(54) **CHROMATOGRAPHIC PROCESSES**
CHROMATOGRAPHIEVERFAHREN
PROCÉDÉS CHROMATOGRAPHIQUES

(30) Priority: 11.08.2009 IN CH19082009
(43) Date of publication of application: 20.06.2012
(73) Proprietor: Biocon Limited, Bangalore 560 100 Karnataka (IN)
(72) Inventor: DAVE, Nitesh, Karnataka 560078 (IN); GULLA, Krishana, Chaitanya, Indore Madhya Pradesh 452001 (IN); SHANKAR, Sundaresh, Karnataka 560047 (IN); IYER, Harish, Bangalore Karnataka 560043 (IN)
(74) Representative: Michalski Hüttermann & Partner Patentanwälte mbB
(86) International application number: PCT/IB2010/053589
(87) International publication number: WO 2011/018745

(56) References cited:
- EP-B1- 0 515 228
- WO-A1-2004/092202
- WO-A1-2007/095661
- US-A- 5 290 920
- D. GUSAROV, V. NEKIPELOVA, V. GUSAROVA, V. LASMAN, D. BAIRAMASHVILI: "Displacement effect during HPLC preparative purification of human insulin", JOURNAL OF CHROMATOGRAPHY B, vol. 877, no. 11-12, 15 April 2009 (2009-04-15), pages 1216-1220, XP026062473,
- ANDREJ FROLOV ET AL: "Separation of Amadori peptides from their unmodified analogs by ion-pairing RP-HPLC with heptafluorobutyric acid as ion-pair reagent", ANALYTICAL AND BIOANALYTICAL CHEMISTRY, SPRINGER, BERLIN, DE, vol. 392, no. 6, 24 September 2008 (2008-09-24), pages 1209-1214, XP019652924, ISSN: 1618-2650, DOI: 10.1007/S00216-008-2377-1
- THOMAS ANDREAS ET AL: "Sensitive and fast identification of urinary human, synthetic and animal insulin by means of nano-UPLC coupled with high-resolution/high-accuracy mass spectrometry", DRUG TESTING AND ANALYSIS, WILEY, US, vol. 1, no. 5, 1 May 2009 (2009-05-01), pages 219-227, XP008160716, ISSN: 1942-7611, DOI: 10.1002/DTA.35 [retrieved on 2009-07-15]
- ROMANCHIKOV A B ET AL.: 'Recombinant Human Insulin. VII. Increased Efficacy of Chromatographic Separation Based on a Principle of Bifunctionality' BIOORGANICHESKAIA KHIMIIA vol. 23, no. 2, February 1997, pages 98 - 103, XP008152155
- KLYUSHNICHENKO V E ET AL.: 'Recombinant Human Insulin. V. Optimization of the Reversed-Phase High-Performance Liquid Chromatographic Separation' JOURNAL OF CHROMATOGRAPHY. B, BIOMEDICAL APPLICATIONS vol. 662, 09 December 1994, pages 363 - 369, XP008152156
- TURNER J G ET AL.: 'Rapid Separation of Tritiated Thyrotropin- Releasing Hormone and its Catabolic Products from Mouse and Human Central Nervous System Tissues by High-Performance Liquid Chromatography with Radioactive Flow Detection' JOURNAL OF CHROMATOGRAPHY vol. 487, 24 February 1989, pages 275 - 286, XP027550778
- PATTHY M ET AL.: 'Purification and Characterization of Peptides with Corticotropin-Releasing Factor Activity from Porcine Hypothalami' PROCEEDINGS OF THE NATIONAL ACADEMY OF SCIENCES OF THE UNITED STATES OF AMERICA vol. 83, May 1986, pages 2969 - 2973, XP008152157
- FROLOV ANDREJ ET AL.: 'Separation of Amadori Peptides from their Unmodified Analogs by lon-pairing RP-HPLC with Heptafluorobutyric acid as Ion-Pair Reagent' ANALYTICAL AND BIOANALYTICAL CHEMISTRY vol. 392, November 2008, pages 1209 - 1214, XP019652924

## Description

### TECHNICAL FIELD:

The present disclosure demonstrates the utility of ion pairing agents in the preparative scale of purification. More particularly, the disclosure relates to the usage of ion pairing agents in reverse phase preparative chromatography enabling high purity of the desired end product. The disclosure shows that ion-pairing agents have dramatic effect on desired purity of polypeptides.

### BACKGROUND AND PRIOR ART OF THE DISCLOSURE:

A number of different chromatographic procedures are applied to obtain the desired end result with respect to purity and yield. Reverse-phase chromatography is one of the most powerful methods of purification employed .Reverse phase liquid chromatography ("RP-LC") and reverse phase high-performance liquid chromatography ("RP-HPLC") are commonly used to purify molecules such as peptides and proteins , produced by either synthetic or recombinant methods. RP-LC and RP-HPLC methods can efficiently separate closely related impurities and have been used to purify many diverse molecules (Lee et al., "Preparative HPLC," 8th Biotechnology Symposium, Pt. 1, 593-610 (1988)). Further, RP-LC and RP-HPLC have been successfully used to purify molecules, particularly; proteins on an industrial scale (Olsen et al., 1994, J. Chromatog. A, 675, 101).

The usage of ion pairing agents such as triethylamine (TEA), Trifluoro acetic acid (TFA), Hexane sulfonic acid (HSA) etc in the analytical method development and its effect on protein peak resolution is well known Shayne Cox Gad; Handbook of Pharmaceutical Biotechnology. An ion pairing agent adsorbs to the stationary phase via its hydrocarbon chain creating an electrical double layer which imposes an electric potential on the surface of the stationary phase. Due to this, the protein/peptide molecules experience both ion exchange with the electrolyte ions of the mobile phase and also the adsorption effect with the reverse phase stationary phase (Frederick F. Cantwell et al., 1984, Journal of Pharmaceutical and Biomedical Analysis, Volume 2, Pages 153-164). Gusarov et al (J.Chromatog. B; 877(2009), pp1216-1220) describe the use of acetic acid and ethanol in the purification of insulin.

The instant disclosure relates to usage of ion pairing agents in the preparative scale of purification of proteins and peptides.

More specifically this disclosure relates to the use of HSA and TEA in RP-HPLC preparative linear chromatography.

The ion pairing agents used in RP-HPLC essentially contain long hydrocarbon chain with an ionisable group. These molecules, in the mobile phase ion pair with the surface charges of the protein/peptide. Due to this ion pairing, the hydrophobicity of the peptide/protein increases which is attributed to the hydrocarbon chain of the ion pairing agent. This interaction depends on the surface charges. Different proteins of a mixture have different surface charges, and hence, bind differentially to the stationary phase which improves the resolution between the protein peaks. The adsorbed ion pairing agents impart a charge specific to its functional group on the surface of the stationary phase. This repels the like charges, thus, changing the selectivity of various proteins in a mixture.

It has been observed in conventional chromatography that as the loading increases on the column the resolution decreases between the impurities and desired protein of interest. The protein bands during elution tend to merge affecting the performance of the chromatography in terms of purity of the preparation and yield. The instant disclosure circumvents this problem which is a key to manufacturability of proteins. Higher loading on the column beyond the protein injected on the column for analytical detection, is crucial for process development and dictates the cost and yield of a given process. The instant disclosure shows that ion pairing agents have dramatic effect in the purity of proteins even after the protein loading was increased. The instant disclosure demonstrates the use of ion pairing in improving yield of the chromatographic step.

### OBJECTIVES OF THE DISCLOSURE

The main objective of the present disclosure is to obtain a chromatographic process for the purification of polypeptides from a mixture.

Another main objective of the present disclosure is to obtain, Insulin analogue such as Aspart, Glargine and Lispro.

Yet another main objective of the present disclosure is to obtain purified polypeptides such as Atosiban and Eptifibatide.

### SUMMARY OF THE DISCLOSURE

Accordingly, the present disclosure relates to a chromatographic process for purification of polypeptide from a mixture having at least one related impurity, said process comprising step of employing RP-HPLC with an ion paring agent having concentration ranging from about 0.01 % to about 2% in combination with organic modifier having concentration ranging from about 5% to about 85 %; a chromatographic process for purification of polypeptide from a mixture having atleast one related impurity, said process comprising steps of a) packing RP-HPLC column with silica (C₄-C₁₈) based resin, equilibrated with about 5% to about 85 % organic modifier, b) loading the polypeptide mixture on the column at a flow rate of about 180 cm/hr to about 360 cm/hr, c) washing the column with an ion pairing agent having concentration ranging from about 0.05% to about 1% in combination with the organic modifier having concentration ranging from about 5% to about 85%, and d) performing a linear gradient of about 10% to about 70% for eluting the purified polypeptide from the column; an Insulin analogue obtained by a process as stated above with purity ranging from about 90% to about 100%; purified Aspart purified with a purity of atleast 98%; purified Glargine purified with a purity of atleast 99%; purified Lispro purified with a purity of atleast 97%; a polypeptide obtained by a process as stated above with purity ranging from about 90% to about 100%, purified Atosiban purified with a purity of atleast 99.14%; and purified Eptifibatide purified with a purity of atleast 94%.

### DETAILED DESCRIPTION OF THE DISCLOSURE

The present disclosure relates to a chromatographic process for purification of polypeptide from a mixture having at least one related impurity, said process comprising step of employing RP-HPLC with an ion paring agent having concentration ranging from about 0.01 % to about 2% in combination with organic modifier having concentration ranging from about 5% to about 85 %.

In an embodiments of the present disclosure most preferably the ion pairing agent and the organic modifier have a concentration ranging from about 0.05% to about 1% and from about 8% to about 65% respectively.

In another embodiment of the present disclosure the ion pairing agent is selected from a group comprising Hexane sulfonic acid, Trifluoro acetic acid, Pentafluoro propanoic acid, Triethyl amine and Heptafluorobutyric acid.

In yet another embodiment of the present disclosure the organic modifier is selected from a group comprising Acetonitrile, Ethanol, Methanol and Isopropyl alcohol.

In still another embodiment of the present disclosure the polypeptide is selected from a group comprising, Insulin Analogue, Eptifibatide and Atosiban.

In still another embodiment of the present disclosure the Insulin analogues are Aspart, Lispro and Glargine.

The present disclosure also relates to a chromatographic process for purification of polypeptide from a mixture having atleast one related impurity, said process comprising steps of: a) packing RP-HPLC column with silica (C₄-C₁₈) based resin, equilibrated with about 5% to about 85 % organic modifier, b) loading the polypeptide mixture on the column at a flow rate of about 180 cm/hr to about 360 cm/hr, c) washing the column with an ion pairing agent having concentration ranging from about 0.05% to about 1% in combination with the organic modifier having concentration ranging from about 5% to about 85%, and d) performing a linear gradient of about 10% to about 70% for eluting the purified polypeptide from the column.

In an embodiment of the present disclosure the silica resin is preferably C₈.

In another embodiment of the present disclosure the chromatographic purification is carried out at a pH ranging from about 2.5 to about 8.5.

In yet another embodiment of the present disclosure the resin has a particle size ranging from about 5µ to about 40µ, preferably, from about 7µ to about 20µ, and most preferably from about 10µ to about 13µ.

In still another embodiment of the present disclosure the resin bead has a pore size ranging from about 50Å to about 2000Å, preferably from about 100Å to about 500Å, and most preferably 120Å.

In still another embodiment of the present disclosure the purity of the polypeptide is ranging from about 90% to about 100%, preferably atleast 99%.

The present disclosure further relates to an Insulin analogue obtained by a process as stated above with purity ranging from about 90% to about 100%.

The present disclosure also relates to purified Aspart purified with a purity of atleast 98%.

The present disclosure also relates to purified Glargine purified with a purity of atleast 99%.

The present disclosure also relates to purified purified Lispro purified with a purity of atleast 97%.

The present disclosure also relates to purified a polypeptide obtained by a process as stated above with purity ranging from about 90% to about 100%.

The present disclosure also relates to purified purified Atosiban purified with a purity of atleast 99.14%.

The present disclosure also relates to purified purified Eptifibatide purified with a purity of atleast 94%.

The disclosure relates to the usage of ion pairing agents such as Triethylamine, Trifluoro acetic acid, Hexane sulfonic acid, Pentafluoro propanoic acid etc in RP-HPLC preparative linear chromatography of polypeptides. More specifically the disclosure relates to the usage of ion pairing agents through reverse phase preparative linear chromatography of Insulin analogues and peptides

Another object and advantage of the present disclosure is increased purity of the desired protein even after the protein loading was increased.

The scope of the present invention is defined by the claims.

### DEFINITION OF TERMS:

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The methods and techniques of the present disclosure are generally performed according to conventional methods well known in the art.

The term 'polypeptide', 'protein', 'peptide' refers to a polymer of amino acids and does not refer to a specific length of the product; thus, peptides, oligopeptides, and proteins are included within the definition of polypeptide. This term also does not refer to or exclude post-expression modifications of the polypeptide although chemical or post-expression modifications of these polypeptides may be included or excluded as specific embodiments. Therefore, for example, modifications to polypeptides that include the covalent attachment of glycosyl groups, acetyl groups, phosphate groups, lipid groups and the like are expressly encompassed by the term polypeptide. Further, polypeptides with these modifications may be specified as individual species to be included or excluded from the present disclosure. In one embodiment, the molecule is a polypeptide or their related analogs or derivatives thereof. According to preferred embodiment, the polypeptide is selected from Insulin analogues such as aspart, lispro and glargine.. Preferably, the polypeptide is a cyclic peptide. According to another preferred embodiment, the polypeptide is a non-cyclic peptide. In still another preferred embodiment, the polypeptide is selected from the group comprising eptifibatide, and , atosiban .

The term "Insulin analog" is intended to encompass any form of "Insulin" as defined above wherein one or more of the amino acids within the polypeptide chain has been replaced with an alternative amino acid and/or wherein one or more of the amino acids has been deleted or wherein one or more additional amino acids has been added to the polypeptide chain. Insulin analogue is selected from the group comprising Aspart, Lispro and Glargine.

Insulin Aspart is a human analogue that is a rapid-acting, parenteral blood glucose-lowering agent. Aspart is homologous with regular human Insulin with the exception of a single substitution of the amino acid proline by aspartic acid in position B28, and is produced by recombinant DNA technology.

Insulin Glargine differs from human Insulin in that the amino acid asparagine at position 21 on the Insulin A-chain is replaced by glycine and two arginines are added to the C-terminus of the B chain. Insulin Glargine is also a human Insulin analogue that is a rapid-acting, parenteral blood glucose-lowering agent.

Insulin Lispro is a human insulin analogue that is a rapid acting, parenteral blood glucose-lowering agent. Chemically, it is Lys (B28), Pro (B29) human insulin analogue, created when the amino acids at positions 28 and 29 on the Insulin B- chain are reversed.

**Atosiban-** a synthetic peptide is an inhibitor of the hormones oxytocin and vasopressin. It is an oxytocin receptor antagonist, is effective in the treatment of an acute episode of preterm labor. It is a competitive antagonist of oxytocin at uterine oxytocin receptors and has been developed as a new tocolytic therapy in the treatment of preterm labour. Atosiban also called ADH (Anti-Diuretic Hormone).

**Eptifibatide-** is is an antiplatelet drug of the glycoprotein IIb/IIIa inhibitor class. Its a cyclic heptapeptide derived from a protein found in the venom of the southeastern pygmy rattlesnake. It belongs to the class of the so called arginin-glycin-aspartat-mimetics and reversibly binds to platelets.

The term "chromatography" refers to the process by which an analyte of interest in a mixture is separated from other solutes in a mixture as a result of differences in rates at which the individual solutes of the mixture migrate through a stationary phase under the influence of a moving phase, or in bind and elute processes.

The term "High Performance liquid chromatography", as used herein, refers to that chromatographic procedure in which the particles (stationary phase) used in the column packing are small (between 3 and 50 microns) and regular with little variation from the selected size. Such chromatography typically employs relatively high (around 500-3500 psi) pressures.

The term "impurity" refers to any product that does not share the same nature as the protein of interest. The impurities are mainly product related. The impurity that has been targeted in the Insulin Aspart crude by using ion pairing agents was Des leader des B arginine Insulin Aspart precursor. This differs from Insulin Aspart by possessing an extension of 5 amino acids at the C-terminal of B chain, the 5 amino acids being Arg-Asp-Ala-Asp-Asp which shows that at acidic pH it has an extra positive charge with respect to Aspart.

"Ion pairing agents" are usually ionic compounds that form ion pairs with oppositely charged ions. Usually ion pairing agents used in RP-HPLC contain a hydrocarbon chain that imparts certain hydrophobicity so that the ion pairing agents can be retained on a reversed-phase column. Typical ion pairing agents capable of forming ion pairs with proteins may be used in the present disclosure which may include Trifluoro Acetic acid (TFA), Hexane sulfonic acid (HSA), Pentafluoro propanoic acid (PFPA), Triethyl amine (TEA), Heptafluorobutyric acid (HFBA), etc.

The ion pairing agents of the invention are defined in the claims.

Specifically this disclosure relates to the use of HSA and TEA in RP-HPLC preparative linear chromatography as exemplified in the preferred embodiments. HSA has a structure composed of a 6C long hydrocarbon chain with a sulfonic acid (SO₃⁻) functional group. In the acidic pH, essentially below the pI value of a peptide/protein, all the functional amino groups of the basic amino acids such as Lysine, Arginine and Histidine present on the surface of the protein structure would be positively charged due to protonation. SO₃⁻ group of HSA exists in the ionic form even at low pH due to the high electron density on the O-atoms which is attributed to the inductive effect shown by the 6-C long hydrocarbon side chain. The SO₃⁻ group would specifically target the positively charged groups on the protein structure. This interaction between HSA and the peptide would bring about a miniscule and reversible conformational change in the protein 3D structure. This would sometimes bring about a change in the selectivity of some impurities during RP chromatography. HSA is very well known for improving the resolution of protein peaks in the analytical RP chromatography. Whereas in the present disclosure, it has been observed that HSA helps in improving the yields and purity levels in preparative chromatography as well.

Triethyl amine (TEA) as a well known ion pairing agent elutes the protein in the form of tight bands and this property is currently employed in higher loading (preparatory loading) to improve yields and purity of the RP chromatography.

"Organic modifiers" are non-polar solvents that are used in RP chromatography which at lower concentrations assist the analyte molecules to bind to the stationary phase and at higher concentrations elute the same. Various organic modifiers are used in the instant disclosure like acetonitrile, isopropyl alcohol, ethanol, methanol, dimethylformamide etc.

The organic modifiers of the invention are defined in the claims.

"Desocta "is a product related impurity that gets generated during the trypsinisation step of the process. It is characterized by lacking 8 amino acids towards the C-terminal of the B-chain of Insulin and its analogues.

0.85RRT, 0.9RRT, 1.05RRT and 1.07RRT are product related impurities that gets generated during reaction intermediates some are generated during process- These impurities are formed because of multiple reaction steps involved in the process of preparation of Atosiban.

0.86RRT, 1.22RRT, 1.8RRT and 2.1RRT are impurities formed because of multiple reaction step involved in the process of preparation of eptifibatide. They are typically reaction intermediates and some are generated during the process. There can be intermediates which have protected groups that prevent them from converting into the product.

The present disclosure relates to the use of ion pairing agents for obtaining substantially pure Insulin analogs and peptide in the range of 90%-100%. Insulin analogues maybe selected from a group comprising of Aspart, Lispro and Glargine.Protein or peptide is Atosiban and eptifibatide.

It is an object of the present disclosure to use at least 0.05% to 1% (w/v) of an ion pairing agent wherein the process is based upon RP-HPLC.Most preferably ion pairing agent is used in the range of 0.05%-1% (w/v).

In a broad aspect, the present disclosure relates to the use of ion pairing being employed for purifying a protein of interest comprising the steps of:
Equilibration to keep the stationary phase ready to bind to the analyte of interest, followed by loading wherein the crude or the impure material containing the analyte is passed through the stationary phase. The sample is loaded at a flow rate of about 180-360 cm/hr. The gradients used are subject to variation with respect the sample peptide to be purified.

The first step of the process herein involves purifying molecules from mixtures containing them by loading the mixtures on a reversed-phase liquid chromatography column. Preferably, the column is packed with a medium having a particle diameter of about 5-40 µ, more preferably about 10-40 µ, and most preferably about 10-13 µ. Preferably, the column has a pore size of about 100-2000 angstroms, more preferably about 100-500 angstroms. In context of the present disclosure the pore size of the resin packed in the column is 120 angstroms.

This is followed by washing to remove the unbound molecules, eluting the bound analyte from the stationary phase and regeneration to remove any tightly bound molecules that does not elute with the given elution conditions. 5%-65%of various organic modifiers are used for equilibration and washing such as acetonitrile, isopropyl alcohol, ethanol, methanol, dimethylformamide etc. The purification is carried out at a pH in the range of 2.5-8.5.

The medium of the column may be any suitable material, including polymeric-based resin media, silica-based media, or methacrylate media.

One aspect of the present disclosure resides in the use of ion pairing agents in RP-HPLC preparative linear chromatography for obtaining substantially pure proteins and peptides. Typical ion pairing agents capable of forming ion pairs with proteins may be used in the present disclosure which may include Trifluoro Acetic acid (TFA), Hexane sulfonic acid (HSA), Pentafluoro propanoic acid (PFPA), Triethyl amine (TEA), Heptafluorobutyric acid (HFBA), Heptafluorobutyric acid (HFBA), etc.

Specifically this disclosure relates to the use of HSA and TEA in RP preparative linear chromatography as exemplified in the preferred embodiments. HSA has a structure composed of a 6C long hydrocarbon chain with a sulfonic acid (SO₃⁻) functional group. In the acidic pH, essentially below the pI value of a peptide/protein, all the functional amino groups of the basic amino acids such as Lysine, Arginine and Histidine present on the surface of the protein structure would be positively charged due to protonation. The SO₃⁻ group would specifically target the positively charged groups on the protein structure. As HSA interacts with the surface charges, the hexyl group of HSA would be exposed on the surface of the protein, which would increase its hydrophobicity. The extent of the interaction of HSA with various peptides would bring about a change in their selectivity. HSA is very well known for improving the resolution of protein peaks in the analytical RP chromatography. Whereas in the present discosure, it has been observed that HSA helps in improving the yields and purity levels in preparative chromatography as well.

Triethyl amine (TEA) also works in the similar way as HSA, TEA ion pairs with the negatively charged moieties on the protein surface and increases the hydrophobicity of the proteins.

Another object of the present disclosure relates to the use of ion pairing agent which helped in changing the selectivity of an impurity which is very closely related to the protein of interest and was a major concern in the purification.

The impurities are mainly product related. The impurity that has been targeted in the Insulin Aspart crude by using ion pairing agents was Des leader des B Arginine Insulin Aspart precursor. This differs from Insulin Aspart by possessing an extension of 5 amino acids at the C-terminal of B chain, the 5 amino acids being Arg-Asp-Ala-Asp-Asp which shows that at acidic pH it has an extra positive charge with respect to Aspart which would be a target for HSA. In Insulin Glargine, HSA helps in separating the Insulin Glargine precursor as it possesses extra positive charges than that of Insulin Glargine.

Yet another aspect of the present disclosure is the increased purity of the desired protein even after the protein loading was increased.

The effective performance of the present disclosure requires the individuation of right combination of the chromatographic matrix to be used, the pH value and the ionic strength of the buffer for efficient purifications.

The pH of the buffer system influences separation combined with hydrophobicity of compounds. The change in pH attributed during different steps of chromatography separation, affect the mobility of compounds in the column.

The foregoing descriptions of specific embodiments of the present disclosure are presented for purposes of illustration and description. They are not intended to be exhaustive or to limit the disclosure to the precise forms disclosed. Various modifications and variations are possible in view of the above teachings. In addition, many modifications may be made to adapt a particular situation, material, composition of matter, process, process step or steps, to the objective, spirit and scope of the present disclosure. All such modifications are intended to be within the scope of the claims appended hereto.

The technology of the instant Application is further elaborated with the help of following examples.

The following examples are provided to further illustrate the embodiments of the present disclosure, but are not intended to limit the scope of the disclosure. While they are typical of those that might be used, other procedures, methodologies, or techniques known to those skilled in the art may alternatively be used.

A more complete understanding can be obtained by reference to the following specific examples, which are provided for purposes of illustration only and are not intended to limit the scope of the disclosure.

### EXAMPLES:

### Control 1

Crude Insulin Aspart material of purity of ∼75% was diluted 10 times with purified water and IPA was added to a final concentration of 5%.Crude mixture was purified on a Kromasil™ (100 Å -13 µ -C8) column. Mobile phase A was 250 mM Sodium acetate, pH 4.0 and the mobile phase B was Isopropyl alcohol. A Gradient Elution of 15% to 18% of mobile phase B in mobile phase A over 20 column volumes. Separation of desocta Insulin Aspart was observed but desleader desB-arginine Insulin Aspart precursor separation was not achieved. The overall purity came to ∼90%.

### EXAMPLE 1

Crude Insulin Aspart material of purity of ∼75% was diluted 10 times with purified water and IPA was added to a final concentration of 5%.Crude mixture was purified on a Kromasil™ (100 Å -13 µ -C8) column. Mobile phase A was 1% hexane Sulfonic acid (HSA) (w/v) in 250 mM Sodium acetate, pH 4.0 and the mobile phase B was Isopropyl alcohol. A Gradient Elution of 18%-22% of mobile phase B in mobile phase A over 20 column volumes. The addition of HSA efficiently removed desocta Insulin Aspart and also reduction of desleader desB-arginine Insulin Aspart precursor from a level of - 2.77% to less than 0.27% was observed and the overall purity achieved is of ∼97.85%.

### Control 2

Crude Insulin Aspart material of purity of ∼75% was diluted 10 times with purified water and ethyl alcohol was added to a final concentration of 10%. Crude mixture was purified on a Kromasil™ (100 Å -13 µ -C8) column. Mobile phase A was 25mM ammonium sulphate in 250 mM sodium acetate, pH 4.0 and the mobile phase B was ethyl alcohol. A gradient elution of 26%-32% of mobile phase B in mobile phase A over 20 column volumes. The desleader desB-arginine Insulin Aspart precursor reduced from a level of ∼2% to 1%. The desocta Insulin Aspart impurity was removed completely and the monoglycosylated Insulin Aspart was reduced from ∼3% to 1.3%. The overall purity achieved is of ∼94%

### EXAMPLE 2

Crude Insulin Aspart material of purity of ∼75% was diluted 10 times with purified water and ethyl alcohol was added to a final concentration of 10%. Crude mixture was purified on a Kromasil™ (100 Å -13 µ -C8) column. Mobile phase A was 1% Hexane Sulfonic acid (HSA) (w/v) in 25mM ammonium sulphate in 250 mM sodium acetate, pH 4.0 and the mobile phase B was ethyl alcohol. A gradient elution of 25%-35% of mobile phase B in mobile phase A over 20 column volumes. The addition of HSA reduced desleader desB-arginine Insulin Aspart precursor from a level of ∼3% to less than 0.3%. The desocta Insulin Aspart was completely removed and the monoglycosylated Insulin Aspart levels was reduced from ∼2% to 0.3%. The overall purity achieved is of ∼98%

### Control 3

Crude Insulin Aspart material of purity of ∼67% was diluted 10 times with purified water and methyl alcohol was added to a final concentration of 10%. Crude mixture was purified on a Kromasil™ (100 Å -13 µ -C8) column. Mobile phase A was 25 mM ammonium sulphate in 250 mM Ammonium acetate, pH 4.0 and the mobile phase B was methyl alcohol. A gradient elution of 45%-55% of mobile phase B in mobile phase A over 20 column volumes. The desleader desB-arginine Insulin Aspart precursor reduced from a level of ∼2% to less than 1%. The monoglycosylated Insulin Aspart impurity did not show any significant reduction. The reduction was from 2% to 1.5%. The desocta Insulin Aspart reduced from ∼10% to less than ∼2%. The overall purity achieved is of ∼89%

### EXAMPLE 3

Crude Insulin Aspart material of purity of ∼67% was diluted 10 times with purified water and methyl alcohol was added to a final concentration of 10%. Crude mixture was purified on a Kromasil™ (100 Å -13 µ -C8) column. Mobile phase A was 1% Hexane Sulfonic acid (HSA) (w/v) in a 25 mM ammonium sulphate in 250 mM Ammonium acetate, pH 4.0 and the mobile phase B was methyl alcohol. A gradient elution of 55%-65% of mobile phase B in mobile phase A over 20 column volumes. The addition of HSA purified desleader desB-arginine Insulin Aspart precursor from a level of ∼2% to less than 1%. The monoglycosylated Insulin Aspart impurity was reduced from 3% to less than 0.5%. The desocta Insulin Aspart reduced from ∼10% to less than ∼2%. The overall purity achieved is of ∼92%

### EXAMPLE 4

Crude Insulin Aspart material of purity of ∼67% was diluted 10 times with purified water and methyl alcohol was added to a final concentration of 10%. Crude mixture was purified on a Kromasil™ (100 Å -13 µ -C8) column. Mobile phase A was 1% hexane sulfonic acid (HSA)(w/v) in 25 mM ammonium sulphate in 250mM ammonium acetate, pH 4.0 and the mobile phase B was methyl alcohol. A gradient elution of 20%-50% of mobile phase B in mobile phase A over 5 column volumes followed by 55%-65% of mobile phase B in mobile phase A over 20 column volumes. The addition of HSA purified desleader desB-arginine Insulin Aspart precursor from a level of ∼2% to less than 1 % and monglycosylated impurity from ∼3% to 0.6%. The desocta Insulin Aspart reduced from ∼10% to less than ∼2%. The overall purity achieved is of ∼92%.

### EXAMPLE 5

Crude Insulin Aspart material of purity of ∼67% was diluted 10 times with purified water and methyl alcohol was added to a final concentration of 10%. Crude mixture was purified on a Kromasil™ (100 Å -13 µ -C8) column. Mobile phase A was 1% Hexane Sulfonic acid (HSA) (w/v) in 25mM ammonium sulphate in 250mM ammonium acetate, pH 3.5 and the mobile phase was methyl alcohol. A gradient elution of 55%-60% of mobile phase B in mobile phase A over 20 column volumes. The addition of HSA purified monglycosylated Insulin Aspart from a level of ∼3% to less than 0.4%. The desocta Insulin Aspart reduced from ∼10% to less than -1%. The overall purity achieved is of ∼90%.

### Control 6

Partially purified Insulin Glargine material of purity of ∼94% was diluted 3 times with purified water and methyl alcohol was added to a final concentration of 10%. Crude mixture was purified on a Kromasil™ (100 Å -13 µ -C8) column. Mobile phase A was 100mM ammonium sulphate, pH 4.0 and the mobile phase B was methyl alcohol. A gradient elution of 51%-59% of mobile phase B in mobile phase A over 20 column volumes. The Insulin Glargine precursor levels were reduced from ∼2.0% to 1.2% and DesB32-R Insulin Glargine was not reduced. The overall purity achieved is of ∼97.5%.

### EXAMPLE 6

Partially purified Insulin Glargine material of purity of ∼94% was diluted 3 times with purified water and methyl alcohol was added to a final concentration of 10%. Crude mixture was purified on a Kromasil™ (100 Å -13 µ -C8) column. Mobile phase A was 0.5% Hexane Sulfonic acid (HSA)(w/v) in 100mM ammonium sulphate, pH 4.0 and the mobile phase B was methyl alcohol. A gradient elution of 51%-59% of mobile phase B in mobile phase A over 20 column volumes. The addition of HSA purified Insulin Glargine precursor from a level of ∼2.5% to less than 0.8% and DesB32-R from 0.7% to less than 0.2%. The overall purity achieved is of ∼99.2%.

### EXAMPLE 7

Partially purified Insulin Lispro material of purity of ∼77% was diluted 3 times with purified water and acetonitrile was added to a final concentration of 10%. Crude mixture was purified on a Kromasil™ (100 Å -13 µ -C8) column. Mobile phase A was 1% Triethyl amine (TEA) (v/v) in 20 mM magnesium chloride and 100mM Tris, pH 8.5 and the mobile phase B was acetonitrile. A gradient elution of 24%-30% of mobile phase B in mobile phase A over 25 column volumes. The addition of TEA purified desleader desB- Arginine Insulin Lispro precursor level of 15% to less than 1%. The overall purity achieved is of ∼97.5%.

### Control 8

Atosiban crude after one step of purification is at a purity of ∼95%. The elution pool of first step is diluted in such a way as to get final solvent concentration to 5%. The load is then purified on a Daiso (120Å- 10µ-C8) column. Mobile phase A was 50mM acetic acid and mobile phase B was Acetonitrile. A gradient elution of 9% to 12% of mobile phase B in mobile phase A over 15 column volumes followed by 12 to 17% mobile phase B in mobile phase A over 10 column volumes was carried out. The purity achieved was ∼96% showing complete removal of only 1.05RRT impurities. The 0.85RRT and 0.9RRT impurity did not show any reduction.

### EXAMPLE 8

Atosiban crude after one step of purification is at a purity of ∼95%. The elution pool of first step is diluted in such a way as to get final solvent concentration to 5%. The load is then purified on a Daiso (120Å- 10µ-C8) column. Mobile phase A was 0.05% hexane sulphonic acid (HSA) (w/v) in 50mM acetic acid and mobile phase B was Acetonitrile. A gradient elution of 10% to 17% of mobile phase B in mobile phase A over 25 column volumes was carried out. The addition of HSA helped in increasing the purity to 98.6% showing complete removal of 0.85RRT, 0.90RRT, 1.05RRT and 1.07RRT impurities.

### Control 9

Atosiban crude after one step of purification is at a purity of ∼95%. The elution pool of first step is diluted in such a way as to get final solvent concentration to 5%. The load is then purified on a Daiso (120Å- 10µ-C8) column. Mobile phase A was 50mM phosphate buffer at pH 8.0 and mobile phase B was Acetonitrile. A gradient elution of 18% to 24% of mobile phase B in mobile phase A over 25 column volumes was carried out. The purity achieved was 98.5%. The 0.97RRT reduced from ∼1.7% to ∼0.6%. The 0.95RRT impurity was not removed, the 1.05RRT and 1.07RRT impurities reduced from ∼0.8% to ∼0.3%.

### EXAMPLE 9

Atosiban crude after one step of purification is at a purity of ∼95%. The elution pool of first step is diluted in such a way as to get final solvent concentration to 5%. The load is then purified on a Daiso (120Å- 10µ-C8) column. Mobile phase A was 0.2% Triethyl amine (TEA) (v/v) in 50mM phosphate buffer at pH 8.0 and mobile phase B was Acetonitrile. A gradient elution of 20% to 24% of mobile phase B in mobile phase A over 25 column volumes was carried out. The addition of TEA helped in increasing the purity to 99.14% showing complete removal of 0.95RRT, 0.97RRT impurities and 50% reduction in 1.05RRT and 1.07RRT impurities.

### EXAMPLE 10:

Crude Eptifibatide is at a purity of ∼58%. Load is prepared by dissolving the crude in 50mM acetic acid and 5% acetonitrile. The load is then purified on a Daiso (120Å-10µ-C8) column. Mobile phase A was 0.1% Trifluro acetic acid (TFA) (v/v) and mobile phase B was Acetonitrile. A gradient elution of 8% to 14% of mobile phase B in mobile phase A over 25 column volumes was carried out. The addition of TFA helped in increasing the purity to 94% showing complete removal of 0.86RRT, 1.22RRT, 1.8RRT and 2.1RRT impurities.

## Claims

1. A chromatographic process for purification of insulin analogue, atosiban or eptifibatide from a mixture having at least one related impurity, at a pH ranging from about 2.5 to about 8.5, said process comprising steps of:
a) packing RP-HPLC column with silica (C₄-C₁₈) based resin, equilibrated with about 5% to about 85 % organic modifier, wherein the organic modifier is selected from a group comprising Acetonitrile, Ethanol, Methanol, Isopropyl alcohol and Dimethylformamide;
b) loading the insulin analogue, atosiban or eptifibatide mixture on the column at a flow rate of about 180 cm/hr to about 360 cm/hr;
c) washing the column with an ion pairing agent having a concentration ranging from about 0.05% to about 1% in combination with the organic modifier having a concentration ranging from about 5% to about 85%, at a pH ranging from about 2.5 to about 8.5, wherein the ion pairing agent is selected from a group consisting of Hexane sulfonic acid, Trifluoro acetic acid, Pentafluoro propanoic acid, Triethyl amine and Heptafluorobutyric acid, and;
d) performing a linear gradient of about 10% to about 70% for eluting the purified insulin analogue, atosiban or eptifibatide from the column.

2. The process as claimed in claim 1, wherein the insulin analogue is selected from a group comprising Aspart, Lispro and Glargine or any combination thereof.

3. The process as claimed in claim 1, wherein the silica resin is C₈.

4. The process as claimed in claim 1, wherein the resin has a particle size ranging from about 5µ to about 40µ.

5. The process as claimed in claim 4, wherein the resin has a particle size ranging from about 7µ to about 20µ.

6. The process as claimed in claim 5, wherein the resin has a particle size ranging from about 10µ. to about 13µ.

7. The process as claimed in claim 1, wherein the resin bead has a pore size ranging from about 50Å to about 2000Å.

8. The process as claimed in claim 7, wherein the resin bead has a pore size ranging from about 100Å to about 500Å.

9. The process as claimed in claim 8, wherein the resin bead has a pore size of 120Å.

10. The process as claimed in claims 1 to 9, wherein purity of the insulin analogue, atosiban or eptifibatide is ranging from about 90% to about 100%, preferably at least 99%.

## Patentansprüche

1. Chromatographisches Verfahren zum Reinigen eines Insulinanalogons, Atosibans oder Eptifibatids aus einem Gemisch mit mindestens einer verwandten Verunreinigung, bei einem pH-Wert im Bereich von etwa 2,5 bis etwa 8,5, wobei das Verfahren die folgenden Schritte aufweist:
a) eine RP-HPLC-Säule mit einem Siliziumdioxid (C₄-C₁₈)-basierten Harz packen, das mit etwa 5% bis etwa 85% organischem Modifizierungsmittel äquilibriert ist, wobei das organische Modifizierungsmittel aus einer Gruppe ausgewählt ist, die Acetonitril, Ethanol, Methanol, Isopropylalkohol und Dimethylformamid umfasst,
b) das Insulinanalogon-, Atosiban- oder Eptifibatidgemisch bei einer Fließgeschwindigkeit von etwa 180 cm/h bis etwa 360 cm/h auf die Säule laden,
c) die Säule mit einem Ionenpaar-Reagenz in einer Konzentration im Bereich von etwa 0,05% bis etwa 1% in Kombination mit dem organischen Modifikationsmittel in einer Konzentration im Bereich von etwa 5% bis etwa 85% bei einem pH-Wert im Bereich von etwa 2,5 bis etwa 8,5 waschen,
wobei das Ionenpaar-Reagenz aus einer Gruppe ausgewählt ist, die aus Hexansulfonsäure, Trifluoressigsäure, Pentafluorpropansäure, Triethylamin und Heptafluorbuttersäure besteht und
d) einen linearen Gradienten von etwa 10% bis etwa 70% durchführen, um das gereinigte Insulinanalogon, Atosiban oder Eptifibatid von der Säule zu eluieren.

2. Verfahren nach Anspruch 1, wobei das Insulinanalogon aus einer Gruppe ausgewählt ist, die Aspart, Lispro und Glargin oder eine beliebige Kombination davon umfasst.

3. Verfahren nach Anspruch 1, wobei das Siliziumdioxidharz C₈ ist.

4. Verfahren nach Anspruch 1, wobei das Harz eine Teilchengröße im Bereich von etwa 5 µm bis etwa 40 µm aufweist.

5. Verfahren nach Anspruch 4, wobei das Harz eine Teilchengröße im Bereich von etwa 7 µm bis etwa 20 µm aufweist.

6. Verfahren nach Anspruch 5, wobei das Harz eine Teilchengröße im Bereich von etwa 10 µm bis etwa 13 µm aufweist.

7. Verfahren nach Anspruch 1, wobei das Harzbett eine Porengröße im Bereich von etwa 50 Å bis etwa 2000 Å aufweist.

8. Verfahren nach Anspruch 7, wobei das Harzbett eine Porengröße im Bereich von etwa 100 Å bis etwa 500 Å aufweist.

9. Verfahren nach Anspruch 8, wobei das Harzbett eine Porengröße von 120 Å aufweist.

10. Verfahren nach einem der Ansprüche 1 bis 9, wobei die Reinheit des Insulinanalogons, Atosibans oder Eptifibatids im Bereich von etwa 90% bis etwa 100%, vorzugsweise wenigstens 99% liegt.

## Revendications

1. Processus chromatographique pour la purification d'analogue d'insuline, d'atosiban ou d'eptifibatide à partir d'un mélange ayant au moins une impureté liée, à un pH dans la plage d'environ 2,5 à environ 8,5, ledit processus comprenant les étapes de :
a) remplissage d'une colonne RP-HPLC avec une résine à base de silice (C₄-C₁₈), équilibrée avec environ 5 % à environ 85 % d'un modificateur organique, dans lequel le modificateur organique est sélectionné à partir d'un groupe comprenant de l'acétonitrile, éthanol, méthanol, alcool isopropylique et diméthylformamide ;
b) chargement du mélange d'analogue d'insuline, d'atosiban ou d'eptifibatide sur la colonne à un débit d'environ 180 cm/h à environ 360 cm/h ;
c) lavage de la colonne avec un agent d'appariement d'ions ayant une concentration dans la plage d'environ 0,05 % à environ 1 % en combinaison avec le modificateur organique ayant une concentration dans la plage d'environ 5 % à environ 85 %, à un pH dans la plage d'environ 2,5 à environ 8,5,
dans lequel l'agent d'appariement d'ions est sélectionné à partir d'un groupe consistant en acide hexanesulfonique, acide trifluoroacétique, acide pentafluoropropanoïque, triéthylamine et acide heptafluorobutyrique ; et
d) exécution d'un gradient linéaire d'environ 10 % à environ 70 % pour dégager l'analogue d'insuline, l'atosiban ou l'eptifibatide purifiés depuis la colonne.

2. Processus selon la revendication 1, dans lequel l'analogue d'insuline est sélectionné à partir d'un groupe comprenant de l'aspart, lispro et glargine ou n'importe quelle combinaison de ceux-ci.

3. Processus selon la revendication 1, dans lequel la résine de silice est du C₈.

4. Processus selon la revendication 1, dans lequel la résine a une taille de particule dans la plage d'environ 5 µm à environ 40 µm.

5. Processus selon la revendication 4, dans lequel la résine a une taille de particule dans la plage d'environ 7 µm à environ 20 µm.

6. Processus selon la revendication 5, dans lequel la résine a une taille de particule dans la plage d'environ 10 µm à environ 13 µm.

7. Processus selon la revendication 1, dans lequel la perle de résine a une taille de pore dans la plage d'environ 50 Å à environ 2000 Å.

8. Processus selon la revendication 7, dans lequel la perle de résine a une taille de pore dans la plage d'environ 100 Å à environ 500 Å.

9. Processus selon la revendication 8, dans lequel la perle de résine a une taille de pore de 120 Å.

10. Processus selon les revendications 1 à 9, dans lequel la pureté de l'analogue d'insuline, de l'atosiban ou de l'eptifibatide est dans la plage d'environ 90 % à environ 100 %, de préférence au moins 99 %.
